# EUROPEAN PATENT APPLICATION

(11) **EP 4 641 479 A1**
(43) Date of publication of application: **29.10.2025**
(21) Application number: 23906599.8
(22) Date of filing: 24.11.2023
(51) Int. Cl.: G06Q 50/12

(54) **FOOD PRODUCT RISK EVALUATION DEVICE, FOOD PRODUCT RISK EVALUATION METHOD, AND FOOD PRODUCT RISK EVALUATION PROGRAM**

(30) Priority: 19.12.2022 JP 2022202477
(71) Applicant: Zensho Holdings Co., Ltd., Minato-ku, Tokyo 108-0075 (JP)
(72) Inventor: KOITABASHI Tsutomu, Tokyo 108-0075 (JP); SERA Kaori, Tokyo 108-0075 (JP)
(74) Representative: AWA Sweden AB
(86) International application number: PCT/JP2023/042253
(87) International publication number: WO 2024/135230

(57) **Abstract**

Provide a food product risk evaluation device, a food product risk evaluation method, and a food product risk evaluation program that enable the user to acquire the details for evaluating food product risk. The food product risk evaluation device includes: a storage that stores first information and second information; a receiver that accepts input regarding the food product to be evaluated for risk and respective details of the multiple risk mitigation elements for the food product; a first acquisition unit that acquires the related elements to be evaluated for the food product on the basis of details of the food product accepted by the receiver and the first information stored in the storage; and a second acquisition unit that acquires inspection items for evaluating food product risk on the basis of hazard elements as food product risk in accordance with the related elements as evaluation targets acquired by the first acquisition unit, the details of the multiple risk mitigation elements accepted by the receiver, and the second information stored in the storage.

## Description

### BACKGROUND OF THE INVENTION

### 1. Field of the Invention

The present disclosure relates to a food product risk evaluation device, a food product risk evaluation method, and a food product risk evaluation program.

### 2. Description of Related Art

In restaurants, inspections are conducted to control food product hygiene and to evaluate the length of time that food product can be consumed safely or with retained quality. For example, the information processing device described in patent document 1 associates the food ingredients with the inspection items needed for the food ingredients and stores them in its storage. Further, the information processing device identifies the food ingredients contained in the dish, refers to the information stored in the storage, and outputs the inspection items in accordance with the identified food ingredients.

### Prior Art Documents

### Patent Documents

Patent Document 1: WO2022/113188

### SUMMARY OF THE INVENTION

Various factors are involved in food risks (food product risks) such as food poisoning. Examples of such factors may include the storage temperature and storage time of the food product. As a specific example here, the food product risk may vary between a case where the food product is stored at 7°C for 5 days and a case where the same food product is stored at 35°C for 6 hours. In addition, the food product risk for the same ingredient may vary between a case where the food product is heated before the dish is consumed and a case where the food product is consumed at room temperature without being heated.

Accordingly, a need for a device that is capable of indicating to restaurant employees and others evaluation details for food product risk, i.e., inspection items related to the food product risk, which vary depending on various factors such as storage and cooking conditions of food products, as in one example described above has been recognized.

The information processing device described in patent document 1 is not capable of providing the risk evaluation details that vary depending on various factors such as the storage and cooking conditions of food products as described above.

This disclosure provides a food product risk evaluation device, a food product risk evaluation method, and a food product risk evaluation program that enable the user to acquire evaluation details for food product risk.

### Means to Solve the Problems

A food product risk evaluation device according to an aspect includes: a storage that stores first information, which associates a food product with related elements related to the food product, and second information, which associates the related elements with risk mitigation elements in accordance with the related elements; a receiver that accepts input regarding the food product to be evaluated for risk and respective details of the multiple risk mitigation elements for the food product; a first acquisition unit that acquires the related elements to be evaluated for the food product on the basis of details of the food product accepted by the receiver and the first information stored in the storage; and a second acquisition unit that acquires inspection items for evaluating food product risk on the basis of hazard elements as food product risk in accordance with the related elements as evaluation targets acquired by the first acquisition unit, the details of the multiple risk mitigation elements accepted by the receiver, and the second information stored in the storage.

### Advantageous Effects of the Invention

According to one aspect, the details to evaluate the food product risk can be acquired.

### BRIEF DESCRIPTION OF THE DRAWINGS

Features, advantages, and technical and industrial significance of exemplary embodiments of the invention will be described below with reference to the accompanying drawings, in which like numerals denote like elements, and wherein:
FIG. 1 shows a diagram for illustrating a food product risk evaluation device according to one embodiment,
FIG. 2 shows a block diagram for illustrating the food product risk evaluation device according to the embodiment,
FIG. 3 shows a list as an example used to illustrate the risk evaluation process, and
FIG. 4 shows a flowchart for illustrating a food product risk evaluation method according to one embodiment.

### DETAILED DESCRIPTION OF EMBODIMENTS

One embodiment will be described below.

### Overview of Food Product Risk Evaluation Device 100

First, an overview of a food product risk evaluation device 100 according to one embodiment will be described.

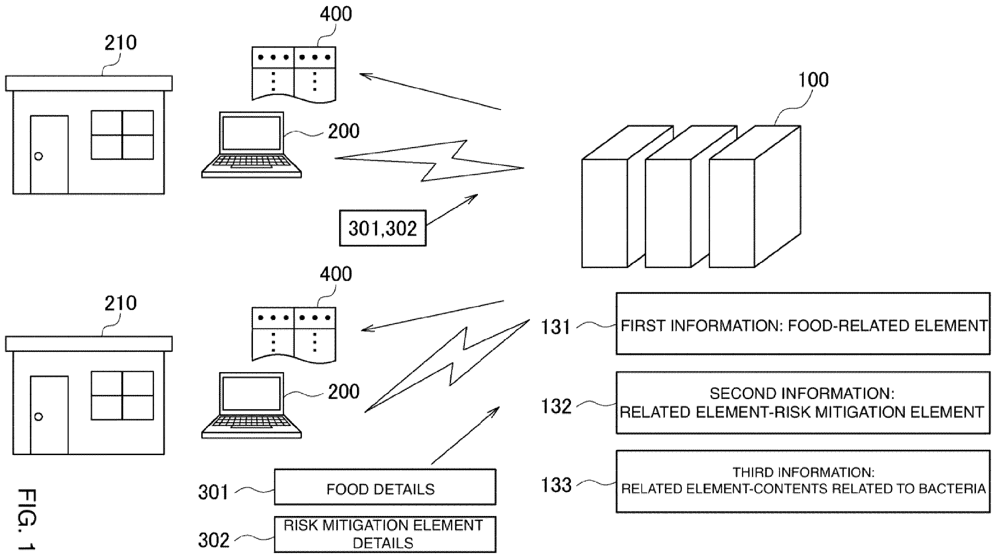

FIG. 1 shows a diagram for illustrating the food product risk evaluation device 100 according to one embodiment.

The food product risk evaluation device 100 may be directed to a computer such as a server, desktop, laptop, tablet and smartphone, for example. In addition, the food product risk evaluation device 100 may be configured, for example, as an information processing device that processes various information related to food product risks.

The food product risk evaluation device 100 may store, for example, the first information 131 and the second information 132. The first information 131 may be directed to, for example, information that associates a food product with related elements related to the food product. The second information 132 may be directed to, for example, information that associates a related element with a plurality of risk mitigation elements in accordance with the related element.

The related elements related to the food product may be various factors, including, for example, the ingredients of the food product, the manufacturing process of the food product, whether the food product is heated before consumption, and the characteristics of the food product (commercial product). As a specific example, if the food product is directed to "pickled cabbage," the related elements may be directed to "cabbage," "chili pepper," and "seasoning liquid," for example.

The related elements may be directed to, for example, the item contents that configure the risk mitigation elements.

Accordingly, an example of the first information 131 may correspond to information that associates the food product "pickled cabbage" with the ingredients "cabbage," "chili pepper," and "seasoning liquid." Another example of the related elements may also correspond to information that associates the food product "pickled cabbage" with the details of the manufacturing process of the food product, whether the food product is heated before consumption, the characteristics of the food product, the storage temperature, and the consumption or expiration date. In the following, "consumption or expiration date" may be referred to as "expiration date."

Depending on the embodiment, the first information 131 may correspond to, for example, information that associates the food product with at least one element selected from a group of the following: the ingredients of the food product, the manufacturing process of the food product, whether the food product is heated before consumption, and the characteristics of the food product (as an example, the storage temperature of the food product, and the expiration date of the food product).

The risk mitigation elements may be directed to, for example, any of the related elements described above that mitigates the risk associated with the food product. Alternatively, the risk mitigation elements may be directed to, for example, the specific details of the related elements that mitigate the food product risk.

As a specific example, the risk mitigation elements may include various details such as: bacteria on the food product is reduced since the food product is disinfected in the manufacturing process, and the increase of bacteria on the food product is less depending on how the food product is stored.

An overview of the process performed by the food product risk evaluation device 100 is as follows.

The food product risk evaluation device 100 acquires the related elements of the food product to be evaluated on the basis of the food product details 301 to be evaluated for risk and the first information 131. As a specific example, if the food product (food product details 301) to be evaluated for risk corresponds to "pickled cabbage," the ingredients of the food product (e.g., "cabbage," "chili pepper" and "seasoning liquid") are acquired as one of the related elements to be evaluated. The food product details 301 may be, for example, the name of the food product, for example, and may be entered using the input unit of the food product risk evaluation device 100 (not shown) or the user terminal 200.

The food product risk evaluation device 100 refers to the third information 133 to acquire the hazard elements in accordance with the related elements. The third information 133 may be directed to, for example, information that associates the related elements with the contents related to the bacteria in accordance with the related elements. As a specific example, in the case where the food product is directed to "pickled cabbage," the ingredients of the food product as one of the related elements are directed to "cabbage," "chili pepper" and "seasoning liquid," and the food product risk evaluation device 100 acquires the risk details (type and number (concentration) of bacteria estimated to be attached to the ingredients) for each of those ingredients.

The food product risk evaluation device 100 also acquires the risk mitigation details that mitigate risk on the basis of the second information 132 and the risk mitigation element details 302.

The risk mitigation element details 302 may be directed to, for example, details entered using the input unit of the food product risk evaluation device 100 (not shown) or the user terminal 200, or details selected from the selection contents recorded in the second information 132. Here, the input unit may be directed to, for example, a keyboard and mouse.

The risk mitigation element details 302 may be various, including, for example, disinfection in the food manufacturing process, a lower (or higher) numerical value (pH value) indicating the acidity (or alkalinity), higher salt content, shorter expiration date of the food product, and heating the food product before consumption, for example.

The food product risk evaluation device 100 refers to the second information 132 to acquire specific mitigation details in accordance with the risk mitigation element details 302, i.e., how much bacteria will be reduced for what type of bacteria, for example. As a specific example, when the food product risk evaluation device 100 receives an input that the disinfection is performed (risk mitigation element details 302) in the risk mitigation element "disinfection in the food manufacturing process," it refers to the second information 132 to acquire specific risk mitigation details such as which type of bacteria is reduced (e.g., the concentration is reduced to 1/10² for type A of bacteria).

The food product risk evaluation device 100 estimates the remaining risk by performing computations on the basis of the acquired hazard elements and risk mitigation details. As a specific example, the food product risk evaluation device 100 may estimate that even if the food product is disinfected in the manufacturing process, type A of bacteria may remain in the food product (residual risk) by the number B of bacteria (predicted concentration).

The food product risk evaluation device 100 may output, for example, type A of bacteria (inspection item 400) for the food product such that inspection for it is conducted. In this case, the food product risk evaluation device 100 may output, for example, the number B of bacteria (predicted concentration) along with the type of bacteria A.

### Details of Food Product Risk Evaluation Device 100

Next, the food product risk evaluation device 100 according to one embodiment will be described in detail.

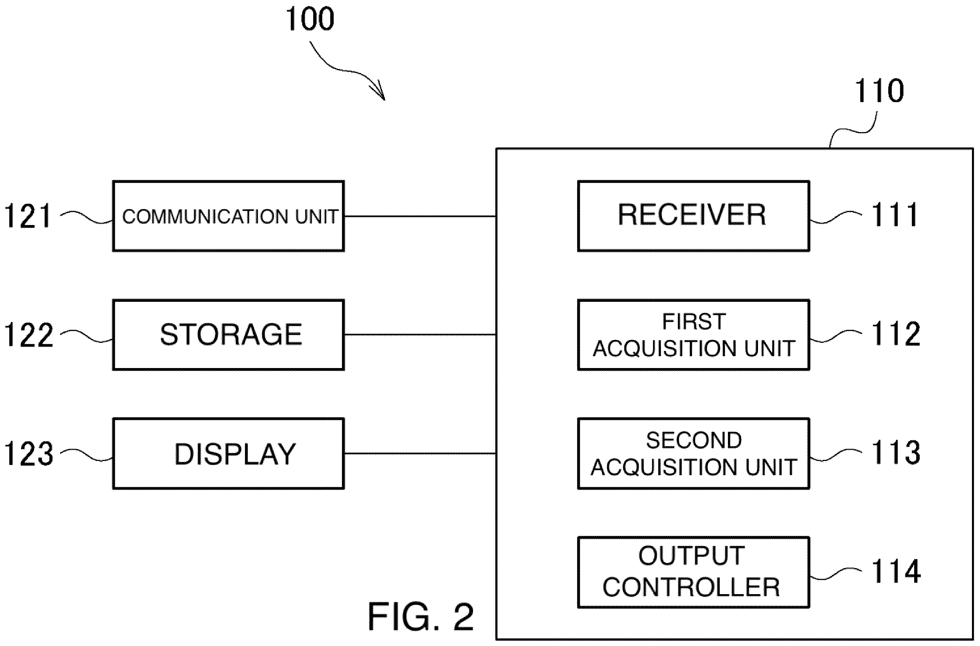

FIG. 2 shows a block diagram for illustrating the food product risk evaluation 100 device according to the embodiment.

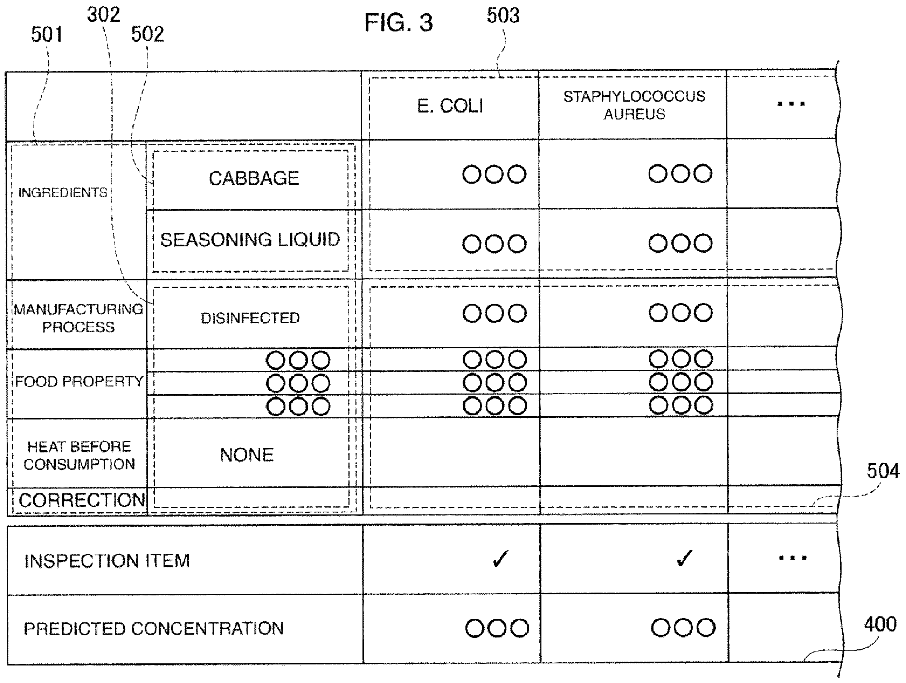

FIG. 3 shows a list as an example used to illustrate the risk evaluation process.

The food product risk evaluation device 100 may include, for example, a communication unit 121, a storage 122, a display 123, and a controller 110. The communication unit 121, storage 122 and display 123 may be a form of an output unit. The controller 110 may include, for example, a receiver 111, a first acquisition unit 112, a second acquisition unit 113, and an output controller 114. The controller 110 may be configured, for example, by the arithmetic processor of the food product risk evaluation device 100. The controller 110 (e.g., arithmetic processor) may embody the functions of units (e.g., the receiver 111, the first acquisition unit 112, the second acquisition unit 113, and the output controller 114) by reading and executing, for example, various programs stored in the storage 122 as appropriate.

The communication unit 121 is directed to a communication interface capable of transmitting and receiving various types of information to and from, for example, devices external to the food product risk evaluation device 100 (external devices). The external devices may be directed to, for example, a user terminal 200 (see FIG. 1) and a server (not shown). The user terminal 200 may be directed to a terminal or other devices used by a user that performs risk evaluation for the food product. The user terminal 200 may be distributed, for example, at facilities 210 (see FIG. 1) where the risk evaluation for the food product is performed, such as restaurants and food factories. The user terminal 200 may be directed to, for example, a desktop, laptop, tablet and smartphone.

The storage 122 may store, for example, various information and programs. An example of the storage 122 may be directed to a memory, solid state drive and hard disk drive. Alternatively, the storage 122 may be directed to, for example, a storage area and server in the cloud.

The storage 122 stores the first information 131, second information 132, and third information 133.

The first information 131 may be directed to, for example, information that associates a food product with related elements related to the food product. The first information 131 may record, for example, ingredients of the food product as the related elements. The first information 131 may also record, for example, at least one of the elements (e.g., items) as the related elements selected from a group of: the manufacturing process of the food product, whether the food product is heated and cooked before consumption, and the characteristics of the food product. The food products recorded in the first information 131 may be directed to, for example, products offered at various facilities 210 such as restaurants and food factories, for example, organizations to which the user of the food product risk evaluation device 100 belongs. The manufacturing process of the food product may be directed to a variety of items, including, for example, whether the food product is disinfected in the manufacturing process. The characteristics of the food product may be directed to a variety of items, including, for example, the storage temperature and expiration date of the food product, whether the food product is acidic (or alkaline), and the salt concentration contained in the food product.

The second information 132 may be directed to, for example, information that associates a related element with a plurality of risk mitigation elements in accordance with the related element. The second information 132 may record, as risk mitigation elements, risk mitigation details for bacteria as a food product risk. The risk mitigation elements may be directed to, for example, any of the related elements described above that mitigates the risk associated with the food product. Alternatively, the risk mitigation elements of the second information 132 may be directed to, for example, specific mitigation details that mitigate a food product risk among the related elements. Examples of specific mitigation details may include various details such as how much the number (concentration) of which type of bacteria is reduced.

The third information 133 may be directed to, for example, information that associates the related elements as hazard elements with the contents related to bacteria in accordance with the related elements. The hazard elements may indicate, for example, how many of which types of bacteria are attached (concentration), for example, in accordance with the related elements. As an example, it may indicate, in accordance with each of the food ingredients as the related elements, how many of which types of bacteria are attached (concentration), for example.

The display 123 may be directed to, for example, a display capable of displaying various characters, symbols, and images.

The receiver 111 accepts input on each of the food product details 301 to be evaluated for risk and the details 302 for each of the multiple risk mitigation elements for the food product.

The receiver 111 may accept, for example, the food product details 301 and the risk mitigation element details 302 entered on the basis of the operation of the input unit (not shown) of the food product risk evaluation device 100. The input unit may be directed to, for example, a keyboard and mouse.

Alternatively, the receiver 111 may accept, for example, the food product details 301 and the risk mitigation element details 302 transmitted from a user terminal 200 or other devices external to the food product risk evaluation device 100 via the communication unit 121.

The food product details 301 may be directed to, for example, the name and abbreviation of the food product.

The risk mitigation element details 302 may be directed to, for example, the specific details of the related elements that mitigate the food product risk. The risk mitigation element details 302 may be phrased, for example, as the contents of the risk mitigation measures. The risk mitigation element details 302 may be directed to, for example, the manufacturing process conditions, characteristics of the food product, whether the food product is heated before consumption, and correction details for the food product subject to risk evaluation.

The manufacturing process conditions may be directed to, for example, various specific details of the food product manufacturing process, such as whether disinfection is performed in the manufacturing process and what the details of the disinfection are.

The characteristics of the food product may be directed to various details such as a numerical value (pH value) indicating the acidity (or alkalinity), salt content, expiration date, and storage temperature of the food product.

The correction details may be directed to, for example, various details when making corrections regarding food product risks (e.g., bacteria causing food poisoning) such as whether the food product corresponds to a fermented food product.

The first acquisition unit 112 acquires the related elements 501 to be evaluated for the food product on the basis of the food product details 301 received by the receiver 111 and the first information 131 stored in the storage 122. That is, the first acquisition unit 112, for example, refers to the first information 131 to acquire the related elements 501 in accordance with the food product to be evaluated for the food product risk. That is, the first acquisition unit 112 may refer to the first information 131 to acquire the related elements 501 to be evaluated in accordance with the food product details 301 received by the receiver 111. Here, the first acquisition unit 112 may, for example, acquire the ingredients 502 in accordance with the food product details 301 as acquiring the related elements 501.

Alternatively, the first acquisition unit 112 may, for example, acquire the related elements 501 in accordance with the food product to be evaluated for the food product risk, which is entered on the basis of the operation of the input unit.

The second acquisition unit 113 may acquire contents 503 related to bacteria in accordance with the related elements 501 as the evaluation targets acquired by the first acquisition unit 112 on the basis of the third information 133 stored in the storage 122. That is, the second acquisition unit 113 may refer to the third information 133 and acquire the type of bacteria and the quantity (concentration) of bacteria (contents 503 related to bacteria) in accordance with the related elements 501 (e.g., ingredients 502) acquired by the first acquisition unit 112. That is, the second acquisition unit 113 may, for example, refer to the third information 133 to acquire the type of bacteria and the quantity (concentration) of bacteria that are estimated to be attached to the food ingredients to be evaluated for the food product risk.

Also, the second acquisition unit 113 associates the risk mitigation element details 302 received by the receiver 111 with some of the related elements 501 acquired by the first acquisition unit 112. The second acquisition unit 113 refers to the second information 132 to acquire the specific mitigation details 504 in accordance with the risk mitigation element details 302.

That is, the second acquisition unit 113 may refer to the second information 132 to acquire the mitigation details 504 that mitigate the food product risk in accordance with the details 302 for the multiple risk mitigation elements received by the receiver 111. That is, the second acquisition unit 113 may, for example, refer to the second information 132 to acquire the reduction in the quantity (concentration) of bacteria in accordance with the type of bacteria after risk mitigation measures are taken for the food product for which food product risk is evaluated. The risk mitigation measures may be directed to specific details of the risk mitigation elements, for example, the manufacturing process, identification of the food product, whether the food product is heated before consumption, and correction details. The reduction in the quantity (concentration) of bacteria in accordance with the type of bacteria may correspond to, for example, mitigation details 504 that mitigate the food product risk.

The second acquisition unit 113 may, for example, acquire the inspection items 400 for the risk evaluation for the food product by computing the "contents 503 related to bacteria" and the "mitigation details 504 that mitigate the food product risk" described above. That is, the second acquisition unit 113 may, for example, acquire the inspection items 400 for the risk evaluation for the food product on the basis of the "contents 503 related to bacteria" and the "mitigation details 504 that mitigate the food product risk" acquired as described above.

Specifically, the second acquisition unit 113 may, for example, reduce the "contents 503 related to bacteria" in accordance with the "mitigation details 504 that mitigate the food product risk" and acquire the inspection items 400 for the risk evaluation for the food product. More specifically, the second acquisition unit 113 subtracts, for example, the mitigation details 504 (type and quantity (concentration) of bacteria to be reduced) that mitigate the food product risk from the contents 503 (type and quantity (concentration) of bacteria) related to bacteria. In this case, the second acquisition unit 113 may acquire the type of bacteria to be inspected and the predicted number of specific bacteria in the food product to be inspected at the time of inspection as the inspect items 400, for example, by computing (e.g., subtracting) the quantity (concentration) of bacteria for each type of bacteria. The "specific bacteria" may be directed to, for example, bacteria that are known to cause food poisoning (e.g., general bacteria, sanitary indicator bacteria such as Escherichia coli, Staphylococcus aureus, and food poisoning bacteria such as Salmonellae genus). The second acquisition unit 113 acquires the inspection items 400 by computing the "contents 504 that mitigate the food product risk" and the "contents 503 related to bacteria," but the operation is not limited to the subtraction, and various operations including multiplication, for example, may be performed.

That is, the second acquisition unit 113 may acquire the inspection items 400 for evaluating the food product risk on the basis of the hazard elements as the food product risk in accordance with the related elements 501 as the evaluation targets acquired by the first acquisition unit 112, the details 302 for the multiple risk mitigation elements received by the receiver 111, and the second information 132 stored in the storage 122.

The output controller 114 may, for example, control the output unit to output the inspection items 400 acquired by the second acquisition unit 113. The output unit may be directed to, for example, the communication unit 121, storage 122 and display 123.

That is, the output controller 114 may, for example, control the communication unit 121 to transmit information on the inspection items 400 to a party external to the food product risk evaluation device 100 (such as the external devices). The external devices may be directed to, for example, a user terminal 200 and a server (not shown).

The output controller 114 may, for example, control the storage 122 to store information on the inspection items 400.

The output controller 114 may, for example, control the display 123 to display the inspection items 400.

### Food Product Risk Evaluation Method

Next, a food product risk evaluation method according to one embodiment will be described.

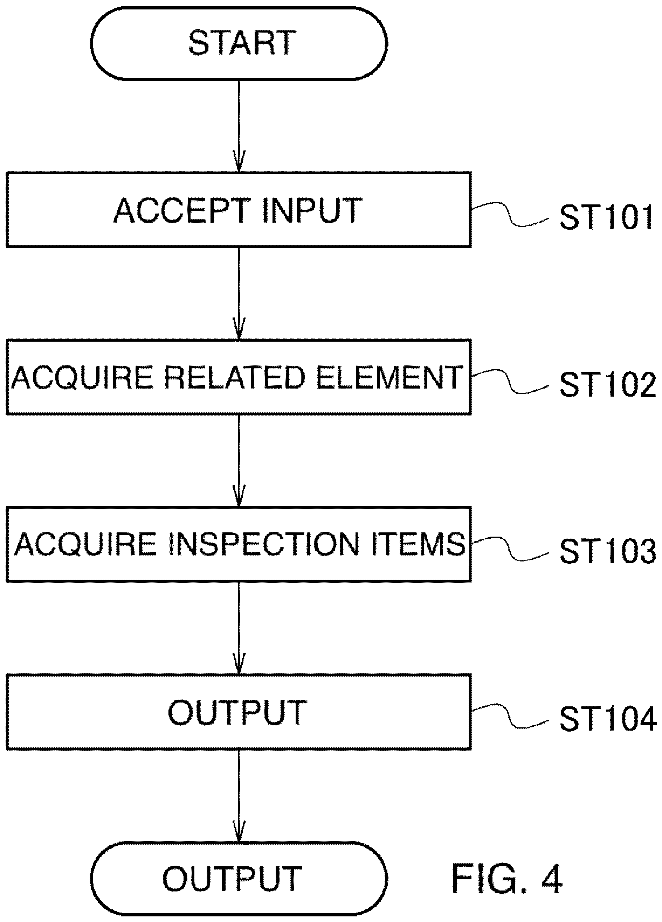

FIG. 4 shows a flowchart for illustrating the food product risk evaluation method according to the embodiment.

In step ST101, the receiver 111 accepts input on the food product details 301 to be evaluated for risk and the details 302 for each of the multiple risk mitigation elements for the food product.

In step ST 102, the first acquisition unit 112 acquires the related elements to be evaluated for the food product on the basis of the food product details 301 received in step ST101 and the first information 131 stored in the storage 122. The first acquisition unit 112 may refer to the first information 131 to acquire the related elements 501 to be evaluated in accordance with the food product details 301 acquired in step ST 101. The related elements here may be directed to elements including, for example, ingredients 502 of the food product (food product details 301). That is, the first acquisition unit 112 accepts the related elements 501 related to the food product details 301.

The first acquisition unit 112 may associate and store the food product details 301 with the acquired related elements 501 and ingredients 502, for example. In this case, the next time or later the food product risk is evaluated, the first acquisition unit 112 may refer to the stored associated information and acquire the related elements 501 and ingredients 502, for example, when the food product details 301 are entered in step ST101.

Alternatively, for example, in step ST101, details including the related elements 501 to be evaluated for the food product and the ingredients 502 of the food product, for example, may be entered as the food product details 301. In this case, the process may proceed to step ST103 without performing the process in step ST102.

In step ST103, the second acquisition unit 113 may acquire the inspection items 400 for evaluating the food risk, on the basis of the hazard elements that can be food product risk in accordance with the related elements 501 as evaluation targets acquired in step ST102, the details 302 for the multiple risk mitigation elements accepted in step ST101, and the second information 132 stored in the storage 122.

Specifically, first, the second acquisition unit 113 may acquire contents 503 related to bacteria in accordance with the related elements 501 as the evaluation targets acquired in step ST102 on the basis of the third information 133 stored in the storage 122. That is, the second acquisition unit 113 acquires the contents of the hazard elements (503) as the food product risk in accordance with the related elements 501.

Next, the second acquisition unit 113 refers to the second information 132 to acquire the mitigation details 504 that mitigate the food product risk in accordance with the details 302 for the multiple risk mitigation elements accepted in step ST101.

The second acquisition unit 113 may then, for example, reduce the "contents 503 related to bacteria" in accordance with the "mitigation details 504 that mitigate the food product risk" and acquire the inspection items 400 for the risk evaluation for the food product. The second acquisition unit 113 may acquire the type of bacteria to be inspected and the predicted number of specific bacteria in the food product to be inspected at the time of inspection as the inspection items 400. That is, the second acquisition unit 113 estimates the food product risk on the basis of the contents (503) of the hazard elements and the risk mitigation element details 302, and acquires the type and predicted number of bacteria to be inspected on the basis of the estimated food product risk. The estimation of the food product risk may be rephrased as, for example, acquiring the inspection items.

In step ST104, the output controller 114 controls the output unit to output the inspection items 400 acquired in step ST103. The output unit may be directed to, for example, the communication unit 121, storage 122 and display 123.

### About Functions and Circuits

Next, the functions and circuits of the food product risk evaluation device 100 described above will be described.

The units of the food product risk evaluation device 100 may be embodied as functions of a computer arithmetic processor or similar device. That is, the receiver 111, first acquisition unit 112, second acquisition unit 113, and output controller 114 (controller 110) of the food product risk evaluation device 100 may be embodied as a reception function, first acquisition function, second acquisition function, and output control function (control function) configuring a computer arithmetic processor or similar device.

A food product risk evaluation program is capable of causing a computer to embody the functions described above. The food product risk evaluation program may be recorded on a non-transitory computer-readable recording medium such as a memory, solid state drive, hard disk drive, or optical disk, for example. A recording medium may be rephrased as, for example, a non-transitory computer-readable medium.

As mentioned above, the units of the food product risk evaluation device 100 may be embodied by a computer arithmetic processor or similar devices. The arithmetic processor or similar devices may be implemented as, for example, an integrated circuit. Accordingly, the units of the food product risk evaluation device 100 may be embodied as circuits configuring the arithmetic processor or similar devices. That is, the receiver 111, first acquisition unit 112, second acquisition unit 113, and output controller 114 (controller 110) of the food product risk evaluation device 100 may be embodied as a reception circuit, first acquisition circuit, second acquisition circuit, and output control circuit (control circuit) configuring the computer arithmetic processor or similar devices.

The communication unit 121, storage 122, and display 123 (output unit) of the food product risk evaluation device 100 may be embodied as communication, storage, and display functions (output function), including, for example, functions of the arithmetic processor or similar devices. The communication unit 121, storage 122, and display 123 (output unit) of the food product risk evaluation device 100 may be embodied as a communication circuit, memory circuit, and display circuit (output circuit) by being implemented as, for example, an integrated circuit. The communication unit 121, storage 122, and display 123 (output unit) of the food product risk evaluation device 100 may be configured as a communication device, storage device, and display device (output device) by being configured by multiple devices, for example.

The food product risk evaluation device 100 can be a combination of one or any number of the multiple units described above.

In this disclosure, the word "information" is used, but the word "information" can be rephrased as "data" and the word "data" can be rephrased as "information."

### Aspects and Effects of this Embodiment

Next, aspects and the effects that each aspect of this embodiment produces will be described. Each aspect described below is an example at the time of application, and the embodiments are not limited to those described below. That is, the embodiments are not limited to each of the aspects described below, but may be embodied by combining the units described above as appropriate. In addition, the lower aspects may be cited by any of the higher aspects.

The effects described below are examples, and the effects produced by each aspect are not limited to those described below. Each aspect may also have at least one of the effects described below, for example.

### (Aspect 1)

A food product risk evaluation device according to an aspect includes: a storage that stores first information, which associates a food product with related elements related to the food product, and second information, which associates the related elements with risk mitigation elements in accordance with the related elements; a receiver that accepts input regarding the food product to be evaluated for risk and respective details of the multiple risk mitigation elements for the food product; a first acquisition unit that acquires the related elements to be evaluated for the food product on the basis of details of the food product accepted by the receiver and the first information stored in the storage; and a second acquisition unit that acquires inspection items for evaluating food product risk on the basis of hazard elements as food product risk in accordance with the related elements as evaluation targets acquired by the first acquisition unit, the details of the multiple risk mitigation elements accepted by the receiver, and the second information stored in the storage.

This allows the food product risk evaluation device to notify the user of the inspection items for the risk evaluation of the food product. The food product risk evaluation device is capable of, for example, notifying the user of the inspection items to ensure that she/he can evaluate the food product risk without relying on the experience of the user to ensure safety of the food product.

### (Aspect 2)

In the food product risk evaluation device according to an aspect, the first information stored in the storage may record, as the related elements, at least one element selected from a group including: ingredients of the food product, a manufacturing process of the food product, whether the food product is heated and cooked before consumption, and characteristics of the food product, and the first acquisition unit may refer to the first information and acquire the related elements to be evaluated in accordance with the details of the food product accepted by the receiver.

This allows the food product risk evaluation device to acquire the inspection items in accordance with various situations related to the food products.

### (Aspect 3)

In the food product risk evaluation device according to an aspect, the storage may store, as third information, hazard elements that associate the related elements with contents related to bacteria in accordance with the related elements, and the second acquisition unit may acquire the contents related to bacteria in accordance with the related elements as evaluation targets acquired by the first acquisition unit on the basis of the third information stored in the storage.

This allows the food product risk evaluation device to acquire the inspection items in accordance with various situations related to the food products.

### (Aspect 4)

In the food product risk evaluation device according to an aspect, the second information stored in the storage may record, as risk mitigation elements, risk mitigation details for bacteria as the food product risk, and the second acquisition unit may acquire mitigation details that mitigate the food product risk in accordance with the details of the multiple risk mitigation elements accepted by the receiver by referring to the second information, reduce the contents related to bacteria in accordance with the acquired mitigation details, and acquire the inspection items for evaluating the food product risk.

This allows the food product risk evaluation device to acquire the inspection items in accordance with various situations related to the food products.

### (Aspect 5)

In the food product risk evaluation device according to an aspect, the second acquisition unit may acquire a type of bacteria to be inspected and a predicted number of specific bacteria in the food product to be inspected at the time of inspection as the inspection items.

This allows the food product risk evaluation device to notify the user of the specific inspection details, thus ensuring that the inspection can be conducted reliably and food safety can be ensured.

### (Aspect 6)

In a food product risk evaluation device according to an aspect, an arithmetic processor that reads and executes a program stored in a storage accepts food product details, accepts related elements related to the food product details, acquires contents of hazard elements as food product risk in accordance with the related elements, acquires details of risk mitigation elements that mitigate the food product risk in accordance with the related elements, and estimates the food product risk on the basis of the contents of the hazard elements and the details of the risk mitigation elements, and acquires a type and predicted number of bacteria to be inspected on the basis of the estimated food product risk.

This allows the food product risk evaluation device to achieve the same effects as those obtained from the aspects described above.

### (Aspect 7)

A food product risk evaluation method according to an aspect causes a computer including a storage that stores first information, which associates a food product with related elements related to the food product, and second information, which associates the related elements with a plurality of risk mitigation elements in accordance with the related elements, to perform: an accepting step of accepting input regarding the food product to be evaluated for risk and respective details of the multiple risk mitigation elements for the food product; a first acquiring step of acquiring the related elements to be evaluated for the food product on the basis of details of the food product accepted in the accepting step and the first information stored in the storage; and a second acquiring step of acquiring inspection items for evaluating food product risk on the basis of hazard elements as food product risk in accordance with the related elements as evaluation targets acquired in the first acquiring step, the details of the multiple risk mitigation elements accepted in the accepting step, and the second information stored in the storage.

This allows the food product risk evaluation method to achieve the same effects as those of the food product risk evaluation device according to the aspects described above.

### (Aspect 8)

A food product risk evaluation program according to an aspect causes a computer to embody: a storage function that stores first information, which associates a food product with related elements related to the food product, and second information, which associates the related elements with multiple risk mitigation elements in accordance with the related elements; an accepting function that accepts input regarding the food product to be evaluated for risk and respective details of the multiple risk mitigation elements for the food product; a first acquisition function that acquires the related elements to be evaluated for the food product on the basis of details of the food product accepted by the accepting function and the first information stored by the storage function; and a second acquisition function that acquires inspection items for evaluating food product risk on the basis of hazard elements as food product risk in accordance with the related elements as evaluation targets acquired by the first acquisition function, the details of the multiple risk mitigation elements accepted by the accepting function, and the second information stored by the storage function.

This allows the food product risk evaluation program to achieve the same effects as those obtained from the food product risk evaluation device according to the aspects described above.

### Description of Reference Numerals

100: Food Product Risk Evaluation Device
110: Controller
111: Receiver
112: First Acquisition Unit
113: Second Acquisition Unit
114: Output Controller
121: Communication Unit
122: Storage
123: Display
200: User Terminal
210: Facility
400: Inspection Item

## Claims

1. A food product risk evaluation device comprising:
a storage that stores first information, which associates a food product with related elements related to the food product, and second information, which associates the related elements with risk mitigation elements in accordance with the related elements;
a receiver that accepts input regarding the food product to be evaluated for risk and respective details of the multiple risk mitigation elements for the food product;
a first acquisition unit that acquires the related elements to be evaluated for the food product on the basis of details of the food product accepted by the receiver and the first information stored in the storage; and
a second acquisition unit that acquires inspection items for evaluating food product risk on the basis of hazard elements as food product risk in accordance with the related elements as evaluation targets acquired by the first acquisition unit, the details of the multiple risk mitigation elements accepted by the receiver, and the second information stored in the storage.

2. The food product risk evaluation device according to claim 1, wherein
the first information stored in the storage records, as the related elements, at least one element selected from a group including: ingredients of the food product, a manufacturing process of the food product, whether the food product is heated and cooked before consumption, and characteristics of the food product, and
the first acquisition unit refers to the first information and acquires the related elements to be evaluated in accordance with the details of the food product accepted by the receiver.

3. The food product risk evaluation device according to claim 1 or 2, wherein
the storage stores, as third information, hazard elements that associate the related elements with contents related to bacteria in accordance with the related elements, and
the second acquisition unit acquires the contents related to bacteria in accordance with the related elements as evaluation targets acquired by the first acquisition unit on the basis of the third information stored in the storage.

4. The food product risk evaluation device according to claim 3, wherein
the second information stored in the storage records, as risk mitigation elements, risk mitigation details for bacteria as the food product risk, and
the second acquisition unit acquires mitigation details that mitigate the food product risk in accordance with the details of the multiple risk mitigation elements accepted by the receiver by referring to the second information, reduces the contents related to bacteria in accordance with the acquired mitigation details, and acquires the inspection items for evaluating the food product risk.

5. The food product risk evaluation device according to claim 4, wherein
the second acquisition unit acquires a type of bacteria to be inspected and a predicted number of specific bacteria in the food product to be inspected at the time of inspection as the inspection items.

6. A food product risk evaluation device comprising:
an arithmetic processor that reads and executes a program stored in a storage, the arithmetic processor
accepts food product details,
accepts related elements related to the food product details,
acquires contents of hazard elements as food product risk in accordance with the related elements,
acquires details of risk mitigation elements that mitigate the food product risk in accordance with the related elements, and
estimates the food product risk on the basis of the contents of the hazard elements and the details of the risk mitigation elements, and acquires a type and predicted number of bacteria to be inspected on the basis of the estimated food product risk.

7. A food product risk evaluation method causing
a computer including a storage that stores first information, which associates a food product with related elements related to the food product, and second information, which associates the related elements with a plurality of risk mitigation elements in accordance with the related elements, to perform:
an accepting step of accepting input regarding the food product to be evaluated for risk and respective details of the multiple risk mitigation elements for the food product;
a first acquiring step of acquiring the related elements to be evaluated for the food product on the basis of details of the food product accepted in the accepting step and the first information stored in the storage; and
a second acquiring step of acquiring inspection items for evaluating food product risk on the basis of hazard elements as food product risk in accordance with the related elements as evaluation targets acquired in the first acquiring step, the details of the multiple risk mitigation elements accepted in the accepting step, and the second information stored in the storage.

8. A food product risk evaluation program causing a computer to embody:
a storage function that stores first information, which associates a food product with related elements related to the food product, and second information, which associates the related elements with multiple risk mitigation elements in accordance with the related elements;
an accepting function that accepts input regarding the food product to be evaluated for risk and respective details of the multiple risk mitigation elements for the food product;
a first acquisition function that acquires the related elements to be evaluated for the food product on the basis of details of the food product accepted by the accepting function and the first information stored by the storage function; and
a second acquisition function that acquires inspection items for evaluating food product risk on the basis of hazard elements as food product risk in accordance with the related elements as evaluation targets acquired by the first acquisition function, the details of the multiple risk mitigation elements accepted by the accepting function, and the second information stored by the storage function.
